(19) Europäisches Patentamt
European Patent Office
Office européen des brevets

(11) Veröffentlichungsnummer: **0 515 796 A1**

# (12) EUROPÄISCHE PATENTANMELDUNG

(21) Anmeldenummer: **92105394.8**

(51) Int. Cl.⁵: **C07D 243/38**, A61K 31/55

(22) Anmeldetag: **28.03.92**

(30) Priorität: **25.05.91 DE 4117123**

(43) Veröffentlichungstag der Anmeldung:
**02.12.92 Patentblatt 92/49**

(84) Benannte Vertragsstaaten:
**AT BE CH DE ES FR GB IT LI NL SE**

(71) Anmelder: **ARZNEIMITTELWERK DRESDEN GmbH**
**Meissner Strasse 35**
**O-8122 Radebeul(DE)**

(72) Erfinder: **Sauer, Wolfgang, Dr.**
**Platanenstrasse 39**
**O-8023 Dresden(DE)**
Erfinder: **Rüger, Carla, Dr.**
**Zillestrasse 7**
**O-8122 Radebeul(DE)**
Erfinder: **Poppe, Hildegard, Dr.**
**Kieler Strasse 6**
**O-8080 Dresden(DE)**

(54) **Neue 5-Aminoacyl-5.10-dihydro-11H-dibenzo/b,e/1,4/diazepin-11-one, Verfahren zu ihrer Herstellung und ihre Verwendung als Arzneimittel.**

(57) 5-Aminoacyl-5,10-dihydro-11H-dibenzo/b,e//1,4/diazepin-11-one der Formel I, worin $R^1$, $R^2$, $R^3$, $R^4$ und n die in der Beschreibung angegebenen Bedeutungen besitzen, haben antiarrhythmische und anticholinerge Eigenschaften und können zum Beispiel durch Umsetzung von 5-Halogenacyl-5,10-dihydro-11H-dibenzo/b,e//1,4/diazepin-11-onen der Formel II mit Aminen der Formel III hergestellt werden.

EP 0 515 796 A1

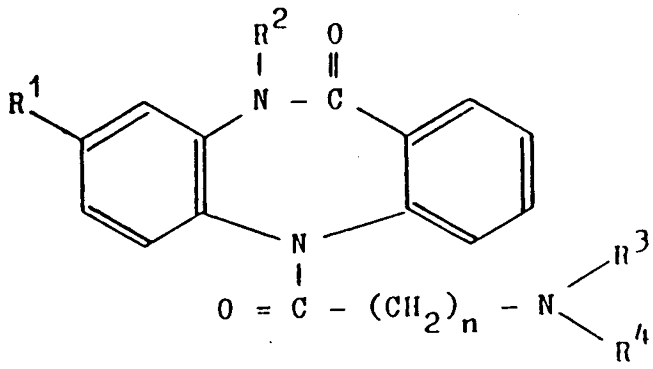

I

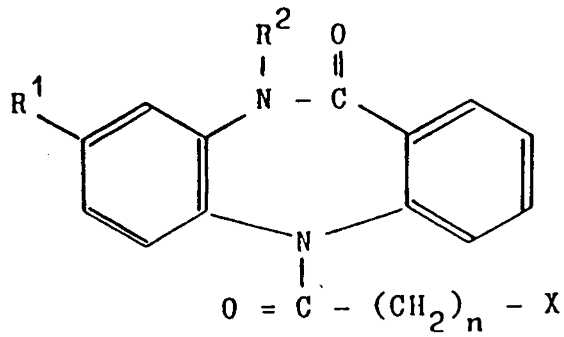

II

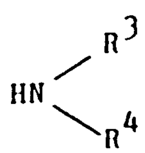

III

Die Erfindung betrifft neue 5-Aminoacyl-10,11-dihydro-11H-dibenzo/b,e//1,4/diazepin-11-one der allgemeinen Formel I, worin $R^1$ Wasserstoff oder Chlor, $R^2$ Methyl, $R^3$ und $R^4$, die gleich oder verschieden sein können und Wasserstoff, geradkettiges oder verzweigtes Alkyl mit 1 bis 6 C-Atomen oder zusammen mit dem N-Atom, an das sie gebunden sind, einen Morpholino- oder N-Methylpiperazinorest und n eine Zahl von 3 bis 6 bedeuten können und wenn $R^3$ Wasserstoff ist, dann kann $R^4$ auch eine gegebenenfalls durch Methyl substituierte Cycloalkyl- oder Cycloalkylmethylgruppe mit 3 bis 7 C-Atomen im Cycloalkylring oder eine gegebenenfalls substituierte Phenylalkylgruppe, worin die Alkylgruppe 1 bis 3 C-Atome enthalten kann und der Phenylring durch Methyl, Methoxy, Halogen oder Trifluormethyl ein- oder zweifach substituiert sein kann und wenn $R^3$ Wasserstoff und $R^4$ eine geradkettige oder verzweigte Alkylgruppe mit 4 bis 6 C-Atomen oder eine der oben genannten Cycloalkyl-, ausgenommen Cyclohexyl selbst, Cycloalkylmethyl- oder Phenylalkylgruppen bedeuten, dann kann $R^2$ auch Wasserstoff sein, ihre Salze mit physiologisch verträglichen anorganischen oder organischen Säuren, Verfahren zu ihrer Herstellung, pharmazeutische Zubereitungen, enthaltend diese Verbindungen und ihre Anwendung als Arzneimittel, insbesondere zur Behandlung von Arrhythmien und cholinergen Störungen.

Es sind bereits 5,10-Dihydro-11H-dibenzo/b,e//1,4/diazepin-11-one bekannt, die in 5-Stellung einen Aminoacetyl- oder einen Aminopropionylrest haben (DE-AS 19 36 670, 20 22 790, 20 65 570, DE-OS 17 95 176, 19 31 487, 27 24 478, 30 28 001, 32 04 157, 32 04 185, BE-PS 753 664, DD-PS 236 731, EP-OS 44 989).

Diese Verbindungen besitzen eine antiulceröse und antisekretorische Wirkung.

Weitere in 5-Stellung mit einem Aminoacetyl- oder Aminopropionylrest substituierte 5,10-Dihydro-11H-dibenzo/b,e//1,4/diazepin-11-one wurden von A. M. Monro und Mitarbeitern in J. Med. Chem. 6, 255 (1963) ohne Angabe pharmakologischer Eigenschaften beschrieben.

Weiterhin sind in 11-Stellung substituierte 5,10-Dihydro-6H-pyrido/2,3-b//1,4/benzodiazepin-6-one mit Einfluß auf die Herzfrequenz bekannt (DE-0S 34 09 237, 35 23 002).

Die meisten Antiarrhythmika der Klasse I wie Lidocain, Ethmozin oder Propafenon haben die Eigenschaft, neben der antiarrhythmischen Wirkung die Herzfrequenz zu senken. Sie sind daher geeignet zur Behandlung von Tachyarrhythmien.

Bei Arrhythmien mit geringer Schlagfolge (Bradyarrhythmien) können diese Mittel deshalb nur in Verbindung mit Atropin zur Anwendung gelangen, damit eine weitere Senkung der Herzfrequenz verhindert wird.

Mittel, die sowohl antiarrhythmisch als auch anticholinerg wirken und damit zur Behandlung von Bradyarrhythmien eingesetzt werden können, sind nur bedingt vorhanden und haben den Nachteil, daß sie entweder überwiegend anticholinerg (Atropin, Ipatropium Bromide) oder überwiegend antiarrhythmisch (Chinidin, Disopyramid) wirken.

In neuester Zeit sind Diazepinone beschrieben worden, die sowohl antiarrhythmische als auch anticholinerge Wirkungen besitzen und damit zur Behandlung von Bradyarrhythmien eingesetzt werden können (EP-0S 326 066 und EP-0S 411 567). Von diesen Verbindungen ist aber noch keine zur klinischen Anwendungsreife gelangt.

Es besteht daher auch weiterhin ein Bedürfnis, Arzneimittel zu finden, die ein möglichst ausgewogenes Verhältnis von antiarrhythmischer und anticholinerger Wirkung besitzen und damit insbesondere zur Therapie von Bradyarrhythmien (Arrhythmien bei langsamer Schlagfolge des Herzens) geeignet sind.

Die vorliegende Erfindung hat die Aufgabe, neue 5-Aminoacyl-5,10-dihydro-11H-dibenzo/b,e//1,4/diazepin-11-one der allgemeinen Formel I, worin $R^1$, $R^2$, $R^3$, $R^4$ und n die oben genannte Bedeutung besitzen, sowie deren Salze mit physiologisch verträglichen anorganischen oder organischen Säuren mit wertvollen pharmakologischen Eigenschaften, insbesondere zur Behandlung von Arrhythmien und cholinergen Störungen, aufzufinden und herzustellen.

Als Beispiele für Verbindungen der allgemeinen Formel I dieser Erfindung seien genannt:

- 5-(6-Dimethylamino-hexanoyl)-10-methyl-5,10-dihydro-11H-dibenzo/b,e//1,4/diazepin-11-on,
- 5-(4-Diethylamino-butyryl)-10-methyl-5,10-dihydro-11H-dibenzo/b,e//1,4/diazepin-11-on,
- 5-(6-Diethylamino-hexanoyl)-10-methyl-5,10-dihydro-11H-dibenzo/b,e//1,4/diazepin-11-on,
- 8-Chlor-5-(4-diethylamino-butyryl)-10-methyl-5,10-dihydro-11H-dibenzo/b,e//1,4/diazepin-11-on
- 8-Chlor-5-(5-diethylamino-valeryl)-10-methyl-5,10-dihydro-11H-dibenzo/b,e//1,4/diazepin-11-on
- 8-Chlor-5-(6-diethylamino-hexanoyl)-10-methyl-5,10-dihydro-11H-dibenzo/b,e//1,4/diazepin-11-on
- 8-Chlor-5-(7-diethylamino-heptanoyl)-10-methyl-5,10-dihydro-11H-dibenzo/b,e//1,4/diazepin-11-on
- 8-Chlor-5-(6-N-morpholino-hexanoyl)-10-methyl-5,10-dihydro-11H-dibenzo/b,e//1,4/diazepin-1.1-on
- 8-Chlor-5-/6-(4-methyl-piperazin-1-yl)-hexanoyl/-10-methyl-5,10-dihydro-11H-dibenzo/b,e//1,4/diazepin-11-on
- 8-Chlor-5-(6-ethylamino-hexanoyl)-10-methyl-5,10-dihydro-11H-dibenzo/b,e//1,4/diazepin-11-on

- 5-(6-sec.-Butylamino-hexanoyl)-5,10-dihydro-11H-dibenzo/b,e//1,4/diazepin-11-on
- 8-Chlor-5-(6-sec.-butylamino-hexanoyl)-5,10-dihydro-11H-dibenzo/b,e//1,4/diazepin-11-on
- 8-Chlor-5-(6-tert.-butylamino-hexanoyl)-5,10-dihydro-11H-dibenzo/b,e//1,4/diazepin-11-on
- 8-Chlor-5-(6-sec.-butylamino-hexanoyl)-10-methy-5,10-dihydro-11H-dibenzo/b,e//1,4/diazepin-11-on
- 8-Chlor-5-/6-(DL-1-phenylethylamino)-hexanoyl/-5,10-dihydro-1H-dibenzo/b,e//1,4/diazepin-11-on
- 8-Chlor-(6-cyclopropylamino-hexanoyl)-5,10-dihydro-11H-dibenzo/b,e//1,4/diazepin-11-on.

Entsprechend der vorliegenden Erfindung können die Verbindungen der allgemeinen Formel I dadurch hergestellt werden, daß man entweder

a) ein 5,10-Dihydro-5-(halogenacyl)-11H-dibenzo/b,e//1,4/diazepin-11-onder allgemeinen Formel II, worin $R^1$, $R^2$ und n die oben genannte Bedeutung besitzen und X ein Halogenatom bedeutet, mit einem Amin der allgemeinen Formel III, in der $R^3$ und $R^4$ die oben genannte Bedeutung besitzen, oder mit einem Salz dieses Amins umsetzt oder daß man

b) ein 5,10-Dihydro-11H-dibenzo/b,e//1,4/diazepin-11-on der allgemeinen Formel IV, worin $R^1$ und $R^2$ die oben genannte Bedeutung besitzen, mit einem Aminoacylhalogenid der allgemeinen Formel V, worin $R^3$, $R^4$ und n die oben genannte Bedeutung besitzen und X' ein Halogenatom, vorzugsweise ein Chlor- oder Bromatom, bedeutet oder mit einem Salz dieses Aminoacylhalogenids umsetzt

und die erhaltenen Verbindungen der allgemeinen Formel I erwünschtenfalls mit physiologisch verträglichen anorganischen oder organischen Säuren in ihre Säureadditionssalze überführt oder aus erhaltenen Salzen der Verbindungen der allgemeinen Formel I erwünschtenfalls die Basen freisetzt.

Eine besondere Ausführungsform der Erfindung besteht darin, daß die Herstellung der Verbindungen der allgemeinen Formel I in einem inerten organischen Lösungsmittel durchgeführt wird. Als inerte organische Lösungsmittel eignen sich zum Beispiel Alkohole wie Ethanol, n-Propanol oder Isopropanol, Ether wie Dioxan oder Tetrahydrofuran, aromatische Kohlenwasserstoffe wie Benzen, Toluen oder Xylen, halogenierte aliphatische oder aromatische Kohlenwasserstoffe wie Chloroform, 1,2-Diohlorethan, Tetrachlorkohlenstoff oder Chlorbenzen oder dipolaraprotische Lösungsmittel wie Dimethylformamid, Acetonitril oder Dimethylsulfoxid.

Eine weitere Ausführungsform der Erfindung besteht darin, daß als Lösungsmittel ein Überschuß der Verbindung der Formel III verwendet wird.

Bei der Herstellung der Verbindungen der allgemeinen Formel I kann die Temperatur in weiten Grenzen variieren. Eine besondere Ausführungsform der Erfindung besteht jedoch darin, daß die Herstellung bei Temperaturen zwischen Raumtemperatur und 150 °C durchgeführt wird.

Entsprechend der vorliegenden Erfindung kann die Herstellung der Verbindungen der allgemeinen Formel I in Gegenwart eines Säureacceptors durchgeführt werden. Als Säureacceptoren eignen sich beispielsweise Alkalikarbonate, Alkalihydrogenkarbonate oder tertiäre organische Amine wie Triethylamin, Pyridin oder N,N-Dimethylanilin.

Die erhaltenen Verbindungen der allgemeinen Formel I können nach an sich bekannten Methoden mit physiologisch verträglichen anorganischen oder organischen Säuren in ihre Säureadditionssalze überführt werden.

Als Beispiele für solche physiologisch verträglichen anorganischen oder organischen Säuren seien genannt: Chlorwasserstoffsäure, Bromwasserstoffsäure, Iodwasserstoffsäure, Schwefelsäure, Phosphorsäure. Essigsäure, Weinsäure, Zitronensäure, Fumarsäure, Maleinsäure, Bernsteinsäure oder Ascorbinsäure.

Ein weiterer Bestandteil der vorliegenden Erfindung besteht in pharmazeutischen Zubereitungen mit einem Gehalt an mindestens einer Verbindung der allgemeinen Formel I oder deren Salzen mit physiologisch verträglichen anorganischen oder organischen Säuren.

Eine besondere Ausführungsform dieses Bestandteiles der Erfindung besteht in pharmazeutischen Zubereitungen zur Behandlung von Arrhythmien, insbesondere von Bradyarrhythmien, mit einem Gehalt an mindestens einer Verbindung der allgemeinen Formel I oder deren Salzen mit physiologisch verträglichen anorganischen oder organischen Säuren.

Eine weitere Ausführungsform dieses Bestandteiles der Erfindung besteht in pharmazeutischen Zubereitungen zur Behandlung von cholinerg induzierten Erkrankungen mit einem Gehalt an mindestens einer Verbindung der allgemeinen Formel I oder deren Salzen mit physiologisch verträglichen anorganischen oder organischen Säuren.

Als pharmazeutische Zubereitungen eignen sich insbesondere Tabletten, Dragees, Kapseln, Lösungen, Ampullen oder Suppositorien mit einem Gehalt an mindestens einer Verbindung der allgemeinen Formel I oder deren Salzen mit physiologisch verträglichen anorganischen oder organischen Säuren, gegebenenfalls im Gemisch mit inerten, pharmazeutisch geeigneten Träger- und/oder Hilfsstoffen. Sie können nach in der pharmazeutischen Praxis allgemein bekannten und üblichen Verfahren hergestellt werden.

Die als Ausgangsverbindungen für das Verfahren b) eingesetzten 5,10-Dihydro-11H-dibenzo/b,e//1,4/diazepin-11-one der allgemeinen Formel IV sind bekannt.

Die bei dem Verfahren a) eingesetzten Ausgangsverbindungen der allgemeinen Formel II lassen sich analog literaturbekannten Methoden durch Umsetzung eines 5,10-Dihydro-11H-dibenzo/b,e//1,4/diazepin-11-on der allgemeinen Formel IV, worin $R^1$ und $R^2$ die oben genannte Bedeutung besitzen, mit einem Halogenacylhalogenid der allgemeinen Formel VI, worin X, X' und n die oben genannte Bedeutung besitzen, gegebenenfalls in Gegenwart halogenwasserstoffbindender Mittel wie Alkalikarbonate oder Alkalihydrogenkarbonate, herstellen.

Diese Umsetzung erfolgt vorzugsweise in einem inerten organischen Lösungsmittel bei erhöhten Temperaturen. Als Lösungsmittel können aromatische Kohlenwasserstoffe wie Benzen, Toluen oder Xylen, halogenierte aromatische Kohlenwasserstoffe wie Chlorbenzen, aber auch Ester wie Essigsäureethylester verwendet werden.

Durch die Erfindung ist es möglich, neue 5-Aminoacyl-5,10-dihydro-11H-dibenzo/b,e//1,4/diazepin-11-one der allgemeinen Formel I sowie deren Salze mit physiologisch verträglichen anorganischen oder organischen Säuren herzustellen.

Die Verbindungen der allgemeinen Formel I sind hochwirksame Herz-Kreislauf-Pharmaka, die eine ausgesprochen starke antiarrhythmische Wirkung besitzen und deshalb zur Therapie von Herzrhythmusstörungen eingesetzt werden können.

Während die meisten Antiarrhythmika wie Lidocain oder Chinidin den Herzrhythmus senken, halten die Verbindungen der allgemeinen Formel I aufgrund der gleichzeitig vorhandenen anticholinergen Eigenschaft den bestehenden Herzrhythmus über längere Zeit auf gleichem Niveau.

Die Kombination von antiarrhythmischer und anticholinerger Wirkung bei den Verbindungen der allgemeinen Formel I ist für die Herstellung von Arzneimitteln von großem Nutzen, die zur Behandlung von bradykarden Rhythmusstörungen eingesetzt werden. Die Verbindungen übertreffen in ihrer Wirkungsstärke im Tierexperiment Präparate wie Chinidin und Lidocain.

Die erfindungsgemäßen neuen 5-Aminoacyl-5,10-dihydro-11H-dibenzo/b,e//1,4/diazepin-11-one der allgemeinen Formel I zeigen bei der tierexperimentellen Prüfung eine ausgeprägte antiarrhythmische Wirkung. In verschiedenen pharmakologischen Modellen zur Prüfung der antiarrhythmischen Wirksamkeit erwiesen sich die erfindungsgemäßen Verbindungen als wesentlich stärker wirksam als Lidocain und Chinidin.

So konnte an der $CaCl_2$-Arrhythmie der Ratte (Methodik bei Femmer, K. und Mitarb., Pharmazie 40, S. 836 (1985)) gegenüber den Vergleichspräparaten Lidocain und Chinidin eine deutlich stärkere antifibrillatorische Wirkung nachgewiesen werden (siehe Tabelle 1).

Die Verbindungen der allgemeinen Formel I zeigen bei intravenöser Anwendung an Ratten eine bessere Verträglichkeit als ihre Referenzpräparate.

Auf Grund der erhöhten Wirkungsstärke und der besseren Verträglichkeit der Verbindungen der allgemeinen Formel I ergibt sich eine wesentliche Vergrößerung der therapeutischen Breite (siehe Tabelle 1, $Q = LD_{50}/ED_{50}$).

Da alle bisher angewandten Antiarrhythmika eine geringe therapeutische Breite besitzen, ist durch die erfindungsgemäßen neuen Verbindungen auch in dieser Hinsicht ein deutlicher Vorteil für die sicherere medizinische Anwendung bei der Behandlung von Herz-Rhythmusstörungen zu erwarten.

Tabelle 1: Antifibrillatorische Wirkung an der $CaCl_2$-Arrhythmie und akute Toxizität an Ratten im Vergleich zu Standardpräparaten

| Verbindung | $CaCl_2$-Arrhythmie Ratte, $ED_{50}$ mg/kg | akute Toxizität Ratte, $LD_{50}$ mg/kg | Q $LD_{50}/ED_{50}$ |
|---|---|---|---|
| Beispiel 4 | 0,033 (0,011 - 0,10) | 78 (73 - 84) | 2364 |
| Beispiel 7 | 0,025 (0,011 - 0,057) | 32 (31 - 34) | 1280 |
| Beispiel 8 | 0,33 (0,21 - 0,51) | 35 (34 - 36) | 106 |
| Chinidin | 2,7 (2,0 - 3,7) | 48 (47 - 49) | 18 |
| Lidokain | 3,1 (1,5 - 6,6) | 18 (17 - 21) | 5,3 |
| Disopyramid | 1,3 (0,83 - 2,2) | m 72 (60 - 80) * | 55 |
| | | w 66 (50 - 110) | 50 |

Werte in Klammern = Vertrauensbereich

* Literaturwert: Prospekt Palpitin (R) der Fa. Gedeon Richter LTD. Budapest / Ungarn, Seite 8, Tabelle 2

Legende zu Tabelle 1:

| | | |
|---|---|---|
| $ED_{50}$ i.v. | = | Dosis in mg/kg, welche die fibrillatorische Wirkung des $CaCl_2$ an Ratten nach i. v. Applikation der Testsubstanz um 50 % unterdrückt. Berechnung nach der Probit-Regressionsanalyse mit Vertrauensbereich, p = 0,05 (Werte in Klammern) |
| $LD_{50}$ i.v. | = | Letale Dosis in mg/kg bei 50 % der Versuchstiere einer orientierenden akuten Toxizität an Wistarratten hauseigener Zucht, i.v. Applikation in die Schwanzvene, 3 - 4 Dosierungen zu je 10 Tieren. Berechnung nach der Probit-Regressionsanalyse mit Vertrauensbereich, p = 0,05 (Werte in Klammern) |
| Q | = | Quotient aus $\frac{LD_{50}\ \text{i.v.}}{ED_{50}\ \text{i.v.}}$ zur Ermittlung der therapeutischen Breite |

Die nachfolgenden Beispiele sollen die Erfindung näher erläutern.

Beispiel 1

8-Chlor-5-(6-tert.-butylamino-hexanoyl)-5,10-dihydro-11H-dibenzo/b,e//1,4/diazepin-11-on.HCl.0,5 $H_2O$

10,5 g (0,025 Mol) 5-(6-Brom-hexanoyl)-8-chlor-5,10-dihydro-11H-dibenzo/b,e//1,4/diazepin-11-on, 35 ml Dimethylformamid und 20 ml tert.-Butylamin werden 15 Stunden unter Rückfluß erhitzt. Anschließend werden tert.-Butylamin und Dimethylformamid abdestilliert.

Der Rückstand wird mit 100 ml Toluen, 30 ml Wasser und 5 ml konzentrierter Ammoniaklösung bis zum Lösen bei 70 bis 80 °C gerührt. Nach dem Abkühlen trennt man die organische Phase ab und rührt die wäßrige Phase mit 10 ml Toluen bei 60 bis 70 °C aus und trennt die toluenische Phase ebenfalls ab.

Die Toluenphasen werden vereinigt und mit 1 g Aktivkohle behandelt. Nach Abtrennen der Aktivkohle wird die Toluenphase zweimal mit 50 ml halbkonzentrierter Salzsäure ausgeschüttelt, die wäßrige salzsaure Phase mit konzentriertem Ammoniak bis pH 10 versetzt und zweimal mit je 50 ml Chloroform extrahiert. Die Chloroformextrakte werden vereinigt und im Vakuum eingeengt. Der Rückstand wird unter Erwärmen in 50 ml Methanol gelöst und die Lösung mit 1 g Aktivkohle versetzt. Nach Abtrennen der Aktivkohle wird in die methanolische Lösung unter Rühren und Kühlen Chlorwasserstoff bis pH 1 eingeleitet. Die Lösung wird im Vakuum eingeengt und der Rückstand in 25 ml Aceton aufgenommen. Nach Stehenlassen über Nacht wird der Niederschlag abgesaugt, mit Aceton gewaschen und bei Raumtemperatur getrocknet.

Zur Reinigung wird das Produkt aus 85 ml Acetonitril umkristallisiert. Die Ausbeute beträgt 5,6 g (48,8 % der Theorie).
Schmelzpunkt: 189 - 196 °C (Zersetzung).

Analyse für $C_{23}H_{30}Cl_2N_3O_{2,5}$
Molmasse: 459,4

| | berechnet | gefunden |
|---|---|---|
| C: | 60,1 % | 59,8 % |
| H: | 6,6 % | 6,4 % |
| Cl: | 15,4 % | 15,5 % |
| N: | 9,1 % | 9,1 % |

Beispiel 2

8-Chlor-5-/6-(DL-1-phenylethylamino)-hexanoyl/-5,10-dihydro-11H-dibenzo/b,e//1,4/diazepin-11-on.HBr

10,5 g (0,025 Mol) 5-(6-Brom-hexanoyl)-8-chlor-5,10-dihydro-11H-dibenzo/b,e//1,4/diazepin-11-on, 100 ml Toluen und 20 ml DL-1-Phenylethylamin werden 8 Stunden bei 130 bis 140 °C gerührt. Nach Erkalten und Stehen über Nacht wird der Niederschlag abgesaugt. Das Filtrat wird im Vakuum eingeengt, der Rückstand in 50 ml Toluen aufgenommen und mit konzentrierter Bromwasserstoffsäure ein pH-Wert von 1 bis 2 eingestellt, wobei sich zunächst eine ölige Phase abscheidet, die beim Stehen kristallisiert. Die Kristalle werden abgesaugt, mit Toluen gewaschen und bei Raumtemperatur getrocknet.

Das Produkt wird mit 150 ml Acetonitril ausgekocht. Der unlösliche Anteil wird abgesaugt und getrocknet. Man erhält 4,2 g (30,9 % der Theorie) vom Schmelzpunkt 170 - 179 °C (Zersetzung).
Analyse für $C_{27}H_{29}BrClN_3O_2$
Molmasse: 542,96

| | berechnet | gefunden |
|---|---|---|
| C: | 59,7 % | 59,8 % |
| H: | 5,4 % | 5,5 % |
| Br: | 14,7 % | 14,8 % |
| N: | 7,7 % | 7,8 % |

Beispiel 3

8-Chlor-5-(6-Diethylamino-hexanoyl)-5,10-dihydro-10-methyl-11H-dibenzo/b,e//1,4/diazepin-11-on.HCl.0,5 $H_2O$

7,4 g (0,019 Mol) 8-Chlor-5-(6-chlor-hexanoyl)-5,10-dihydro-10-methyl-11H-dibenzo/b,e//1,4/diazepin-11-on werden in 30 ml Dimethylformamid mit 30 ml (21,1 g ≙ 0,29 Mol) Diethylamin 10 Stunden am Rückfluß gerührt. Das Lösungsmittel wird im Vakuum abdestilliert, der Rückstand zwischen 30 ml Chloroform und 1 : 1 verdünnter Ammoniaklösung verteilt und die Chloroformphase viermal mit 15 ml konzentrierter Salzsäure ausgeschüttelt. Die salzsauren Phasen versetzt man mit 50 ml Chloroform, tropft langsam konzentrierte Ammoniaklösung bis zu einem pH-Wert von 10 zu, rührt gut durch, trennt das Chloroform ab, extrahiert nochmals mit Chloroform, trocknet die Chloroformphasen und engt ein.

Den Rückstand löst man in Aceton, leitet HCl-Gas bis pH 1 ein, kocht mit Aktivkohle aus und engt das Filtrat ein. Der verbleibende Rückstand wird bei 0,2 Torr und 61 °C getrocknet. Die Ausbeute beträgt 30 % der Theorie (bezogen auf 8-Chlor-5,10-dihydro-10-methyl-11H-dibenzo/b,e//1,4/diazepin-11-on). Das Produkt weist hygroskopische Eigenschaften auf.
Analyse für $C_{24}H_{32}N_3O_{2,5}Cl_2$
Molmasse: 473,45

| | berechnet | gefunden |
|---|---|---|
| C: | 60,89 % | 60,24 % |
| H: | 6,81 % | 6,84 % |
| N: | 8,88 % | 8,92 % |
| Cl: | 14,89 % | 15,33 % |
| $H_2O$ | 1,94 % | 2,00 % |

Beispiel 4

5-(6-Diethylamino-hexanoyl)-5,10-dihydro-10-methyl-11H-dibenzo/b,e//1,4/diazepin-11-on.HCl.0,5 $H_2O$

7,8 g (0,02 Mol) 5-(6-Chlor-hexanoyl)-5,10-dihydro-1o-methyl-11H-dibenzo/b,e//1,4/diazepin-11-on werden in 30 ml Dimethylformamid mit 30 ml Diethylamin (21,1 g ≙ 0,29 Mol) 10 Stunden am Rückfluß gerührt. Das Reaktionsgemisch wird anschließend im Vakuum eingeengt, der Rückstand zwischen 30 ml Toluen, 5 ml konzentrierter Ammoniaklösung und 20 ml Wasser verteilt, die wäßrige Phase abgetrennt und zweimal mit Toluen extrahiert. Die toluenischen Phasen werden vereinigt und dreimal mit 5 ml 1 : 1 verdünnter HCl ausgeschüttelt.

Die salzsauren Phasen werden mit konzentrierter Ammoniaklösung basisch (pH-Wert 10) gestellt und mit Chloroform extrahiert. Nachdem man die Chloroformphase getrocknet und mit Aktivkohle behandelt hat, wird sie eingeengt. Den Rückstand löst man in 20 ml Aceton, leitet HCl-Gas (pH 1) ein und destilliert das Aceton ab. Das erhaltene Hydrochlorid ist hygroskopisch und wird im Hochvakuum über $P_2O_5$ getrocknet. Die Ausbeute beträgt 26 % der Theorie.
Analyse für $C_{24}H_{33}N_3O_{2,5}Cl$
Molmasse: 439,00

| | berechnet | gefunden |
|---|---|---|
| C: | 65,66 % | 65,12 % |
| H: | 7,58 % | 7,55 % |
| N: | 9`57 % | 9,53 % |
| Cl: | 8,08 % | 8,11 % |
| $H_2O$: | 2,05 % | 1,5 % |

Beispiel 5

8-Chlor-5-(6-cyclopropylamino-hexanoyl)-5,10-dihydro-11H-dibenzo/b,e//1,4/diazepin-11-on.HCl.0,5 $H_2O$

4,2 g (0,01 Mol) 8-Chlor-5-(6-brom-hexanoyl)-5,10-dihydro-11H-dibenzo/b,e//1,4/diazepin-11-on und 5 ml Cyclopropylamin werden 4 Stunden am Rückfluß erhitzt. Anschließend wird das Reaktionsgemisch zwischen 30 ml Chloroform und 10 ml 1 : 1 verdünnter Ammoniaklösung verteilt. Die Chloroformphase wird einmal mit 10 ml Wasser und dreimal mit 10 ml konzentrierter Salzsäure ausgeschüttelt. Die vereinigten salzsauren Phasen wurden mit 5 ml Chloroform ausgeschüttelt und anschließend mit konzentrierter Ammoniaklösung pH 11 eingestellt. Man extrahiert anschließend dreimal mit 20 ml Chloroform, schüttelt die vereinigten Chloroformphasen einmal mit Wasser aus, trocknet mit $Na_2SO_4$ und engt im Vakuum zur Trockne ein. Den Rückstand löst man in der Hitze in 10 ml Aceton und leitet in die noch warme Lösung HCl-Gas ein. Das ausgefallene Hydrochlorid wird aus 9 ml Isopropanol und 5 ml Acetonitril umkristallisiert. Die Ausbeute beträgt 36 % der Theorie. Schmelzpunkt: 135 °C (Zersetzung).
Analyse für $C_{22}H_{26}N_3O_{2,5}Cl_2$
Molmasse: 452,4

| | berechnet | gefunden |
|---|---|---|
| C: | 59,60 % | 59,61 % |
| N: | 9,48 % | 9,49 % |
| Cl: | 15,99 % | 15,72 % |
| $H_2O$: | 2,03 % | 1,90 % |

Beispiel 6

8-Chlor-5-(6-sec.-butylamino-hexanoyl)-5,10-dihydro-11H-dibenzo/b,e//1,4/diazepin-11-on.HCl.$H_2O$

8,4 g (0,02 Mol) 8-Chlor-5-(6-brom-hexanoyl)-5,10-dihydro-11H-dibenzo/b,e//1,4/diazepin-11-on und 50 ml sec.-Butylamin werden 3 Stunden am Rückfluß gerührt. Das Reaktionsgemisch engt man anschließend ein, verteilt den Rückstand zwischen 50 ml Chloroform und 20 ml 1 : 1 verdünnter Ammoniaklösung, schüttelt die Chloroformphase einmal mit 20 ml Wasser und dreimal mit 20 ml konzentrierter HCl aus. Die vereinigten salzsauren Phasen werden einmal mit 10 ml Chloroform ausgeschüttelt, mit konzentrierter Ammoniaklösung versetzt und dreimal mit 25 ml Chloroform extrahiert. Die vereinigten getrockneten Chloroformphasen engt man ein, löst den Rückstand in 50 ml Isopropanol, kocht mit Aktivkohle aus, leitet in die warme Lösung HCl-Gas ein, engt ein, erhitzt den Rückstand in 50 ml Aceton und trennt das ungelöste Hydrochlorid ab. Das Hydrochlorid wird aus 30 ml Methanol unter Zusatz von Aktivkohle umkristallisiert. Die Ausbeute beträgt 38,4 % der Theorie. Schmelzpunkt: 150 °C (Zersetzung).
Analyse für $C_{23}H_{31}N_3O_3Cl_2$
Molmasse: 468,42

| | berechnet | gefunden |
|---|---|---|
| C: | 58,97 % | 58,47 % |
| H: | 6,67 % | 6,31 % |
| N: | 8,97 % | 8,83 % |
| Cl: | 15,14 % | 14,92 % |
| $H_2O$: | 3,8 % | 3,2 % |

Beispiel 7

5-(6-Cyclohexylamino-hexanoyl)-5,10-dihydro-10-methyl-11H-dibenzo/b,e//1,4/diazepin-11-on.HCl

13,5 g (0,03 Mol) 5-(6-Iod-hexanoyl)-5,10-dihydro-10-methyl-11H-dibenz/b,e//1,4/diazepin-11-on, 7,35 ml (0,075 Mol) Cyclohexylamin und 60 ml Toluen werden 8 Stunden am Rückfluß erhitzt. Das Reaktionsgemisch wird anschließend mit 10 ml 1 : 1 verdünnter Ammoniaklösung, zweimal mit 20 ml Wasser und dreimal mit 20 ml konzentrierter HCl ausgeschüttelt. Die vereinigten salzsauren Phasen stellt man mit konzentrierter Ammoniaklösung basisch, schüttelt mehrmals mit Chloroform aus und engt die Chloroformphasen nach dem Ausschütteln mit Wasser und Trocknen ein. Der Rückstand wird in 50 ml Aceton aufgenommen, HCl-Gas eingeleitet und das ausgefallene Hydrochlorid aus Ethanol umkristallisiert. Die Ausbeute beträgt 59 % der Theorie. Schmelzpunkt: 238 - 243 °C.
Analyse für $C_{26}H_{34}N_3O_2Cl$
Molmasse: 456,03

| | berechnet | gefunden |
|---|---|---|
| C: | 68,48 % | 68,00 % |
| H: | 7,52 % | 7,55 % |
| N: | 9,21 % | 9,24 % |
| Cl: | 7,77 % | 7,70 % |

Beispiel 8

8-Chlor-5-(6-cyclohexylamino-hexanoyl)-5,10-dihydro-10-methyl-11H-dibenzo/b,e//1,4/diazepin-11-on.HCl

4,83 g (0,01 Mol) 8-Chlor-5-(6-iod-hexanoyl)-5,10-dihydro-10-methyl-11H-dibenzo/b,e//1,4/diazepin-11-on, 3 g (0,03 Mol) Cyclohexylamin und 10 ml Toluen werden 2 Stunden auf 80 °C erwärmt. Anschließend schüttelt man mit 10 ml Wasser und zweimal mit 10 ml konzentrierter HCl aus, versetzt mit Chloroform und konzentrierter Ammoniaklösung und trennt die Chloroformphase ab. Sie wird getrocknet, mit Aktivkohle behandelt, eingeengt, der Rückstand in Aceton gelöst und HCl Gas bis pH 1 eingeleitet. Nach dem Einengen erhält man eine schwach hygroskopische Substanz mit einer Ausbeute von 31 % der Theorie.

Beispiel 9

8-Chlor-5-/6-(4-chlorbenzylamino)-hexanoyl/-5,10-dihydro-11H-dibenzo/b,e//1,4/diazepin-11-on.0.75 $H_2O$

12,63 g (0,03 Mol) 8-Chlor-5-(6-bromhexanoyl)-5,10-dihydro-11H-dibenzo/b,e//1,4/diazepin-11-on, 10,6 g (0,075 Mol) 4-Chlor-benzylamin und 80 ml Toluen werden 10 Stunden am Rückfluß erhitzt. Das Reaktionsgemisch wird anschließend zweimal mit 100 ml Wasser ausgeschüttelt, nötigenfalls filtriert. Das durch Stehenlassen über Nacht ausgefallene Produkt wird abgesaugt, in einem Gemisch von 100 ml destillierten Wasser und 100 ml Chloroform unter Rühren gelöst, mit wenig 1 : 1 verdünnter Ammoniaklösung pH 9 - 10 eingestellt und 30 Minuten bei 40 °C gerührt.

Anschließend werden die Phasen getrennt und die wäßrige Phase nochmals mit 50 mi Chloroform ausgeschüttelt. Die vereinten Chloroformphasen werden mit 100 ml Wasser extrahiert, über Natriumsulfat und Supranoritkohle 30 Minuten stehen gelassen, filtriert und das Chloroform abdestilliert. Der Rückstand wird aus Acetonitril umkristallisiert. Die Ausbeute beträgt 1,7 g (12 % der Theorie). Schmelzpunkt: 153 - 156 °C.

Analyse für $C_{26}H_{26,5}N_3O_{2,75}Cl_2$
Molmasse: 495,92

| | berechnet | gefunden |
|---|---|---|
| C: | 62,96 % | 62,91 % |
| H: | 5,38 % | 5,79 % |
| N: | 8,47 % | 8,66 % |
| Cl: | 14,29 % | 14,87 % |

Beispiel 10

8-Chlor-5,10-dihydro-11H-10-methyl-5-/6-(4-methylpiperazino)-hexanoyl/-dibenzo/b,e//1,4/diazepin-11-on.2 $H_2O$

10,5 g (0,025 Mol) 8-Chlor-5-(6-bromhexanoyl)-5,10-dihydro-10-methyl-11H-dibenz/b,e//1,4/diazepin-11-on gelöst in 45 ml Toluen und 25 g (0,25 Mol) 1-Methylpiperazin werden 2 Stunden am Rückfluß erhitzt. Anschließend werden unter Vakuum überschüssiges 1-Methylpiperazin und Toluen abdestilliert. Der Rückstand wird in 90 ml Chloroform gelöst, mit 15 ml 1 : 1 verdünnter Ammoniaklösung und 25 ml Wasser, zweimal mit 20 ml Wasser und dreimal mit 15 ml konzentrierter Salzsäure ausgeschüttelt. Die vereinigten salzsauren Phasen werden einmal mit 15 ml Chloroform extrahiert, mit 40 ml Wasser verdünnt, mit 50 ml Chloroform unterschichtet und mit wenig konzentrierter Ammoniaklösung auf pH 9 - 10 eingestellt und ausgeschüttelt. Die wäßrige Phase wird noch dreimal mit mit je 15 ml Chloroform extrahiert. Die vereinigten Chloroformphasen engt man nach dem Ausschütteln mit Wasser, Trocknen und Ausrühren mit 1 g Kohle ein. Der zähflüssige Rückstand wird in 50 ml trockenem Aceton gelöst, HCl-Gas eingeleitet, das Aceton im Vakuum abdestilliert, erneut in 50 ml absolutem Ethanol gelöst, mit 1 g Kohle verrührt, filtriert und das Ethanol unter Vakuum abdestilliert. Der kristalline Rückstand wird durch präparative HPLC (Si 60, MeOH/AcCN = 20/80) gereinigt. Die Ausbeute beträgt 8,5 g (60,3 % der Theorie). Schmelzpunkt: 159 - 166 °C.

Analyse für $C_{25}H_{37}N_4O_4Cl_3$
Molmasse: 563,89

| | berechnet | gefunden |
|---|---|---|
| C: | 53,24 % | 53,26 % |
| H: | 6,61 % | 7,03 % |
| N: | 9,94 % | 9,96 % |
| Cl: | 18,86 % | 18,26 % |

Beispiel 11

8-Chlor-5,10-dihydro-11H-5-/6-(2-phenylethylamino)-hexanoyl/dibenzo/b,e//1,4/diazepin-11-on. 0,5 $H_2O$

12,63 g (0,03 Mol) 8-Chlor-5-(6-bromhexanoyl)-5,10-dihydro-11H-dibenzo/b,e//1,4/diazepin-11-on, 9,1 g (0,075 Mol) 2-Phenylethylamin und 80 ml Toluen werden 4 Stunden am Rückfluß erhitzt. Das Reaktionsgemisch wird anschließend zweimal mit 100 ml Wasser ausgeschüttelt. Unter Vakuum wird das Toluen abdestilliert. Der ölige Rückstand wird in 20 ml trockenem Aceton warm gelöst und filtriert. In das Filtrat wird trockenes HCl-Gas eingeleitet, der nach einiger Zeit ausgefallene Niederschlag abgesaugt und mit 20 ml Aceton gewaschen. Die Reinigung erfolgt durch dreimalige Umkristallisation aus Acetonitril unter Zusatz von jeweils 0,8 g, 0,4 g bzw. 0,1 g Supranoritkohle. Die Ausbeute beträgt nach vollständiger Reinigung 4,4 g (29 % der Theorie). Schmelzpunkt: 164 - 170 °C.
Analyse für $C_{27}H_{30}N_3O_{2,5}Cl_2$
Molmasse: 507,47

| | berechnet | gefunden |
|---|---|---|
| C: | 63,99 % | 64,05 % |
| H: | 5,95 % | 5,99 % |
| N: | 8,28 % | 8,25 % |
| Cl: | 13,97 % | 13,98 % |

Beispiel 12

8-Chlor-5,10-dihydro-11H-5-/6-(3-phenylpropylamino)-hexanoyl/-dibenzo/b,e//1,4/diazepin-11-on.HCl . 0,4 $H_2O$

12,63 g (0,03 Mol) 8-Chlor-5-6-bromhexanoyl)-5,10-dihydro-11H-dibenzo/b,e//1,4/diazepin-11-on, 10,44 g (0,075 Mol) 3-Phenyl-propylamin und 80 ml Toluen werden 7 Stunden am Rückfluß erhitzt. Das Reaktionsgemisch wird anschließend zweimal mit 100 ml Wasser ausgeschüttelt. Die toluenische Phase wird unter Vakuum eingeengt, der Rückstand in 50 ml Acetonitril gelöst und trockenes HCl-Gas eingeleitet. Der Niederschlag wird abgesaugt, in 200 ml Chloroform und 300 ml Wasser unter kräftigem Schütteln gelöst, mit Ammoniaklösung pH 7 eingestellt, extrahiert, die wäßrige Phase mit 50 ml Chloroform ausgeschüttelt, die vereinten Chloroformphasen zweimal mit destilliertem Wasser extrahiert, die Chloroformphasen über Natriumsulfat und Kohle stehen gelassen, filtriert und das Chloroform abdestilliert. Der Rückstand wird aus Acetonitril umkristallisiert. Die Ausbeute beträgt 4,8 g (31 % der Theorie). Schmelzpunkt: 152 - 157 °C.
Analyse für $C_{28}H_{31}N_3O_{2,4}Cl$
Molmasse: 519,69

| | berechnet | gefunden |
|---|---|---|
| C: | 64,70 % | 64,83 % |
| H: | 6,17 % | 6,15 % |
| N: | 8.08 % | 7,93 % |
| Cl: | 13,64 % | 13,52 % |

Beispiel 13

8-Chlor-5-(6-cycloheptylamino-hexanoyl)-5,10-dihydro-11H-dibenzo/b,e//1,4/diazepin-11-on.HCl . 0,3 $H_2O$

12,63 g (0,03 Mol) 8-Chlor-5-(6-bromhexanoyl)-5,10-dihydro-11H-dibenzo/b,e//1,4/diazepin-11-on, 11,32 g (0,075 Mol) Cycloheptylamin und 80 ml Toluen werden 3 Stunden am Rückfluß erhitzt. Nach dem Erkalten wird das Reaktionsgemisch einmal mit 150 ml und einmal mit 100 ml Wasser ausgeschüttelt. Die toluenische Phase wird im Vakuum eingeengt, der ölige Rückstand in 70 ml trockenem Acetonitril warm gelöst und trockenes HCl-Gas bis pH 1 eingeleitet. Der ausgefallene Niederschlag wird abgesaugt, in 300 ml Wasser und 200 ml Chloroform unter kräftigem Schütteln suspendiert und erneut abgesaugt. Durch Einengen der Chloroformphase wird weiteres Produkt erhalten. Die vereinigten Niederschläge werden aus Acetonitril / Ethanol = 10 : 1 umkristallisiert. Die Ausbeute beträgt 7,3 g (49 % der Theorie). Schmelzpunkt: 180 - 186 °C.

Analyse für $C_{26}H_{32,6}N_3O_{2,6}Cl_2$

Molmasse: 494,89

| | berechnet | gefunden |
|---|---|---|
| C: | 63,09 %, | 63,07 % |
| H: | 6,64 % | 6,81 % |
| N: | 8,49 % | 8,49 % |
| Cl: | 14,33 % | 14,39 % |

**Patentansprüche**

1. Neue 5-Aminoacyl-5,10-dihydro-11H-dibenzo/b,e//1,4/diazepin-11-one der allgemeinen Formel I, worin $R^1$ Wasserstoff oder Chlor, $R^2$ Methyl, $R^3$ und $R^4$, die gleich oder verschieden sein können und Wasserstoff, geradkettiges oder verzweigtes Alkyl mit 1 bis 6 C-Atomen oder zusammen mit dem N-Atom, an das sie gebunden sind, einen Morpholino- oder N-Methylpiperazinorest und n eine Zahl von 3 bis 6 bedeuten können und wenn $R^3$ Wasserstoff ist, dann kann $R^4$ auch eine gegebenenfalls durch Methyl substituierte Cycloalkyl- oder Cycloalkylmethylgruppe mit 3 bis 7 C-Atomen im Cycloalkylring oder eine gegebenenfalls substituierte Phenylalkylgruppe, worin die Alkylgruppe 1 bis 3 C-Atome enthalten kann und der Phenylring durch Methyl, Methoxy, Halogen oder Trifluormethyl ein- oder zweifach substituiert sein kann und wenn $R^3$ Wasserstoff und $R^4$ eine geradkettige oder verzweigte Alkylgruppe mit 4 bis 6 C-Atomen oder eine der oben genannten Cycloalkyl-, ausgenommen Cyclohexyl selbst, Cycloalkylmethyl- oder Phenylalkylgruppen bedeuten, dann kann $R^2$ auch Wasserstoff sein.

2. Salze der Verbindungen der allgemeinen Formel I in Anspruch 1 mit physiologisch verträglichen anorganischen oder organischen Säuren.

3. Die Verbindungen
   - 5-(6-Dimethylamino-hexanoyl)-10-methyl-5,10-dihydro-11H-dibenzo/b,e//1,4/diazepin-11-on
   - 5-(4-Diethylamino-butyryl)-10-methyl-5,10-dihydro-11H-dibenzo/b,e//1,4/diazepin-11-on
   - 5-(6-Diethylamino-hexanoyl)-10-methyl-5,10-dihydro-11H-dibenzo/b,e//1,47diazepin-11-on
   - 8-Chlor-5-(4-diethylamino-butyryl)-10-methyl-5,10-dihydro-11H-dibenzo/b,e//1,4/diazepin-11-on
   - 8-Chlor-5-(5-diethylamino-valeryl)-10-methyl-5,10-dihydro-11H-dibenzo/b,e//1,4/diazepin-11-on
   - 8-Chlor-5-(6-diethylamino-hexanoyl)-10-methyl-5,10-dihydro-11H-dibenzo/b,e//1,4/diazepin-11-on
   - 8-Chlor-5-(7-diethylamino-heptanoyl)-10-methyl-5,10-dihydro-11H-dibenzo/b,e//1,4/diazepin-11-on
   - 8-Chlor-5-(6-N-morpholino-hexanoyl)-10-methyl-5,10-dihydro-11H-dibenzo/b,e//1,4/diazepin-11-on
   - 8-Chlor-5-/6-(4-methyl-piperazin-1-yl)-hexanoyl/-10-methyl-5,10-dihydro-11H-dibenzo/b,e//1,4/diazepin-11-on
   - 8-Chlor-5-(6-ethylamino-hexanoyl)-10-methyl-5,10-dihydro-11H-dibenzo/b,e//1,4/diazepin-11-on
   - 5-(6-sec.-Butylamino-hexanoyl)-5,10-dihydro-11H-dibenzo/b,e//1,4/diazepin-11-on
   - 8-Chlor-5-(6-sec.-butylamino-hexanoyl)-5,10-dihydro-11H-dibenzo/b,e//1,4/diazepin-11-on

- 8-Chlor-5-(6-tert.-butylamino-hexanoyl)-5,10-dihydro-11H-dibenzo/b,e//1,4/diazepin-11-on
- 8-Chlor-5-(6-sec.-butylamino-hexanoyl)-10-methyl-5,10-dihydro-11H-dibenzo/b,e//1,4/diazepin-11-on
- 8-Chlor-5-/6-(DL-1-phenylethylamino)-hexanoyl/-5,10-dihydro-11H-dibenzo/b,e//1,4/diazepin-11-on
- 8-Chlor-5-(6-cyclopropylamino-hexanoyl)-5,10-dihydro-11H-dibenzo/b,e//1,4/diazepin-11-on

**4.** Verfahren zur Herstellung von 5-Aminoacyl-5,10-dihydro-11H-dibenzo/b,e//1,4/diazepin-11-onen der allgemeinen Formel I, worin $R^1$, $R^2$, $R^3$, $R^4$ und n die oben genannten Bedeutungen besitzen sowie ihrer Salze mit physiologisch verträglichen anorganischen oder organischen Säuren, dadurch gekennzeichnet, daß man entweder

a) ein 5,10-Dihydro-5-halogenacyl-11H-dibenzo/b,e//1,4/diazepin-11-on der allgemeinen Formel II, worin $R^1$, $R^2$ und n die oben genannten Bedeutungen besitzen und X ein Halogenatom bedeutet, mit einem Amin der allgemeinen Formel III, in der $R^3$ und $R^4$ die oben genannten Bedeutungen besitzen, oder mit einem Salz dieses Amine umsetzt, oder daß man

b) ein 5,10-Dihydro-11H-dibenzo/b,e//1,4/diazepin-11-on der allgemeinen Formel IV, worin $R^1$ und $R^2$ die oben genannten Bedeutungen besitzen, mit einem Aminoacylhalogenid der allgemeinen Formel V, worin $R^3$, $R^4$ und n die oben genannten Bedeutungen besitzen und X' ein Halogenatom, vorzugsweise ein Chlor- oder Bromatom, bedeutet oder mit einem Salz dieses Aminoacylhalogenids umsetzt

und die erhaltenen Verbindungen der allgemeinen Formel I erwünschtenfalls mit physiologisch verträglichen anorganischen oder organischen Säuren in ihre Säureadditionssalze überführt oder aus erhaltenen Salzen der Verbindungen der allgemeinen Formel I erwünschtenfalls die Basen freisetzt.

**5.** Verfahren nach Anspruch 4, dadurch gekennzeichnet, daß die Umsetzungen in einem inerten organischen Lösungsmittel durchgeführt werden.

**6.** Verfahren nach den Ansprüchen 4 und 5, dadurch gekennzeichnet, daß als indifferente organische Lösungsmittel Alkohole wie Ethanol, n-Propanol oder Isopropanol, Ether wie Dioxan oder Tetrahydrofuran, aromatische Kohlenwasserstoffe wie Benzen, Toluen oder Xylen, halogenierte aliphatische oder aromatische Kohlenwasserstoffe wie Chloroform, 1,2-Dichlorethan, Tetrachlorkohlenstoff oder Chlorbenzen oder dipolar-aprotische Lösungsmittel wie Dimethylformamid, Acetonitril oder Dimethylsulfoxid verwendet werden.

**7.** Verfahren nach Anspruch 4a, dadurch gekennzeichnet, daß als Lösungsmittel ein Überschuß der Verbindung der allgemeinen Formel III verwendet wird.

**8.** Verfahren nach den Ansprüchen 4 bis 7, dadurch gekennzeichnet, daß die Umsetzungen bei Temperaturen zwischen Raumtemperatur und 150 °C durchgeführt werden.

**9.** Verfahren nach den Ansprüchen 4 bis 8, dadurch gekennzeichnet, daß die Umsetzungen in Gegenwart eines Säureacceptors durchgeführt werden.

**10.** Verfahren nach den Ansprüchen 4 bis 9, dadurch gekennzeichnet, daß als Säureacceptoren Alkalikarbonate, Alkalihydrogenkarbonate oder N,N-Dimethylanilin verwendet werden.

**11.** Pharmazeutische Zubereitungen, gekennzeichnet durch einen Gehalt an mindestens einer Verbindung der allgemeinen Formel I aus Anspruch 1 oder deren Salzen mit physiologisch verträglichen anorganischen oder organischen Säuren.

**12.** Pharmazeutische Zubereitungen zur Behandlung von Arrhythmien, gekennzeichnet durch einen Gehalt an mindestens einer Verbindung der allgemeinen Formel I aus Anspruch 1 oder deren Salzen mit physiologisch verträglichen anorganischen oder organischen Säuren.

**13.** Pharmazeutische Zubereitungen zur Behandlung von Bradyarrhythmien, gekennzeichnet durch einen Gehalt an mindestens einer Verbindung der allgemeinen Formel I aus Anspruch 1 oder deren Salzen mit physiologisch verträglichen anorganischen oder organischen Säuren.

**14.** Pharmazeutische Zubereitungen zur Behandlung von cholinerg induzierten Erkrankungen, gekennzeichnet durch einen Gehalt an mindestens einer Verbindung der allgemeinen Formel I aus Anspruch 1 oder deren Salzen mit physiologisch verträglichen anorganischen oder organischen Säuren.

**15.** Verfahren zur Herstellung von pharmazeutischen Zubereitungen gemäß der Ansprüche 11 bis 14, dadurch gekennzeichnet, daß man Verbindungen der allgemeinen Formel I aus Anspruch 1 oder deren Salze mit physiologisch verträglichen anorganischen oder organischen Säuren zusammen mit inerten, nicht toxischen, pharmazeutisch geeigneten Träger- und/oder Hilfsstoffen verwendet.

**16.** Verwendung von Verbindungen der allgemeinen Formel I in Anspruch 1 oder deren Salzen mit physiologisch verträglichen anorganischen oder organischen Säuren zur Behandlung von Arrhythmien und/oder cholinerg induzierten Erkrankungen.

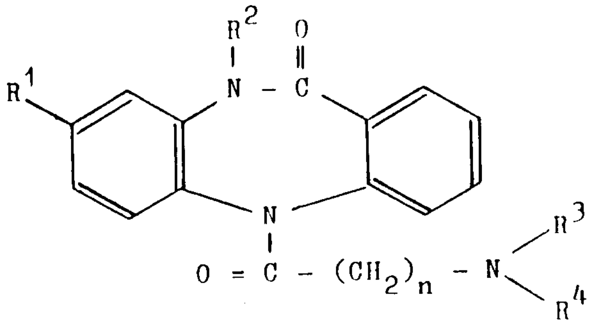

I

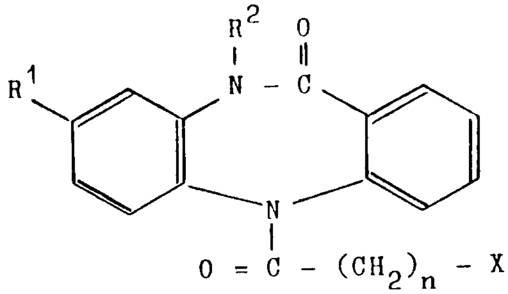

II

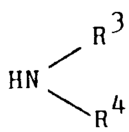

III

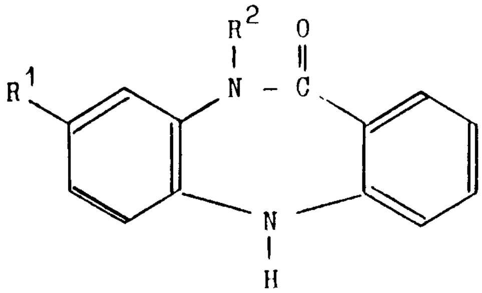

IV

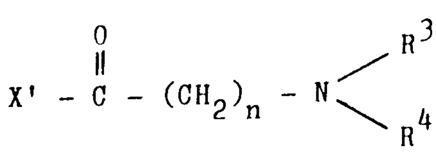

V

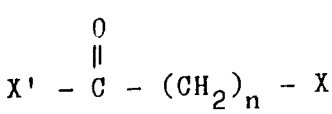

VI

Europäisches Patentamt

# EUROPÄISCHER RECHERCHENBERICHT

Nummer der Anmeldung

EP 92 10 5394

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 411 567 (ARZNEIMITTELWERK DRESDEN GMBH I.G.)<br>* Ansprüche 1,5,7-9 * | 1,2,4, 11-13,15 | C07D243/38<br>A61K31/55 |
| D,Y | EP-A-0 326 066 (DR. K. THOMAE GMBH)<br>* Ansprüche 1,5-10; Beispiele 3,10 * | 1,2,4, 11-13,15 | |
| P,X | WO-A-9 110 654 (PFIZER LTD.)<br>* Ansprüche 1,4,5; Seiten 13 - 15, Beispiele 9 und 10 * | 1,2,11 | |
| A<br>D | EP-A-0 139 627 (VEB ARZNEIMITTELWERK DRESDEN)<br>* Ansprüche 1-8 *<br>& DD-A-236 731 | 1,4-10 | |
| A,D | DE-A-3 409 237 (DR. K. THOMAE GMBH)<br>* Ansprüche 1,8,9; Seite 52, letzter Absatz bis Seite 54, letzte Zeile * | 1,11-14 | |
| A,D | DE-A-3 523 002 (DR. K. THOMAE GMBH)<br>* Ansprüche 1,9 * | 1,11,13 | RECHERCHIERTE SACHGEBIETE (Int. Cl.5)<br>C07D |

Der vorliegende Recherchenbericht wurde für alle Patentansprüche erstellt

| Recherchenort | Abschlußdatum der Recherche | Prüfer |
|---|---|---|
| BERLIN | 12 JUNI 1992 | Christian Hass |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)